# EUROPEAN PATENT SPECIFICATION

(11) **EP 4 499 807 B1**
(45) Date of publication and mention of the grant of the patent: **06.05.2026**
(21) Application number: 23715161.8
(22) Date of filing: 30.03.2023
(51) Int. Cl.: C12N 1/14, D06P 1/34, C12R 1/77

(54) **ISOLATED FUSARIUM SOLANI IIA AND ITS USE FOR DYEING SUBSTRATES**
ISOLIERTER FUSARIUM SOLANI IIA UND DESSEN VERWENDUNG ZUM FÄRBEN VON SUBSTRATEN
SOLANI FUSARIUM IIA ISOLÉ ET SON UTILISATION POUR COLORER DES SUBSTRATS

(30) Priority: 31.03.2022 EP 22166139; 23.08.2022 EP 22191719
(43) Date of publication of application: 05.02.2025
(73) Proprietor: VTL GmbH, 1030 Wien (AT)
(72) Inventor: FLECK, Karin, 1030 Wien (AT); RAUSCHER, Mascha, 1140 Wien (AT); SCHOPF, Erich, 1220 Wien (AT)
(74) Representative: Clark, Claudia
(86) International application number: PCT/EP2023/058376
(87) International publication number: WO 2023/187100

(56) References cited:
- RATHNA JANARTHANAM ET AL: "Production of naphthoquinones and phenolics by a novel isolate Fusarium solani PSC-R of Palk Bay and their industrial applications", BIORESOURCE TECHNOLOGY, vol. 213, 1 August 2016 (2016-08-01), AMSTERDAM, NL, pages 289 - 298, XP093014740, ISSN: 0960-8524, DOI: 10.1016/j.biortech.2016.04.050
- MENEZES BRUNA S. ET AL: "Pigment production by Fusarium solani BRM054066 and determination of antioxidant and anti-inflammatory properties", vol. 10, no. 1, 1 December 2020 (2020-12-01), XP093014625, Retrieved from the Internet <URL:https://www.ncbi.nlm.nih.gov/pmc/articles/PMC7329961/pdf/13568_2020_Article_1054.pdf> DOI: 10.1186/s13568-020-01054-y
- MOLELEKOA TUMISI BEIRI JEREMIAH ET AL: "Production of Pigments by Filamentous Fungi Cultured on Agro-Industrial by-Products Using Submerged and Solid-State Fermentation Methods", FERMENTATION, vol. 7, no. 4, 2 December 2021 (2021-12-02), pages 295, XP093014674, DOI: 10.3390/fermentation7040295

## Description

### FIELD OF THE INVENTION

The invention relates to the field of fungal production of dyes and pigments and applying said fungal production of dyes for dyeing substrates. More specifically, the invention relates to an isolated fungus belonging to the species *Fusarium solani,* a dye produced by said fungus, a method for dyeing a substrate using said fungus, a method for dyeing a substrate using the dye produced by said fungus, a production method of said dye, and also further applications of said dye as an antimicrobial substance.

### BACKGROUND OF THE INVENTION

The textile industry is one of the largest global industrial polluters and has one of the largest water footprints. The dyeing process is one of the main culprits of polluting rivers and lakes, posing a working hazard for textile workers and eventually the end consumers. Estimations reveal the use of 79 billion cubic meters of water within the global textile and clothing industry in 2015 corresponding to one third of EU's whole economy need in 2017.

Slama HB et al (2021) review synthetic dyes for the textile industry, their discharge impact and treatment methods. Dyes are used for colorization of different types of substrates such as textile fibers, paper, cosmetics, but also for food and pharmaceutical products. The textile industry alone accounts for ~75% of the global dye market and involves around ten thousand different dyes. Textile industries produce fibers to form yarn, which is converted to fabric. Different types of dyeing processes are used for dyeing textile materials such as coating the textile uniformly with the dye, printing of a dye in a specific area of the textile material, bleaching, and finishing comprising crosslinking, softening, and waterproofing the textile material. Two main categories of dyes are known: natural dyes which are derived mainly from plants, and synthetic dyes which are artificially synthesized from chemical compounds. Synthetic dyes are further classified into cellulose fiber dyes such as reactive dyes, direct dyes, indigo dyes, and sulfur dyes; protein fiber dyes such as azo dyes, anthraquinone dyes, triarylmethane dyes, and phthalocyanine dyes, and synthetic fiber dyes such as disperse dyes and basic dyes.

As described by Slama HB et al (2021), synthetic dyes are mainly derived from petrochemical compounds and are commercialized in liquid, powder, pastes, or granule forms. The majority of these synthetic dyes cause harmful impacts when discharged in non-treated or partially treated forms in the environment and cause multi-contamination effects on air, soil, plants, and water resources, but they also cause severe human diseases.

Furthermore, the production of synthetic dyes from petrochemical compounds has a substantial impact on the environment because of the extensive and expansive environmental impact of the petrochemical industry.

The production of pigments and dyes by microorganisms as well as the subsequent method of dyeing are the promising alternatives for a "greener" and sustainable dyeing industry.

Kristensen SB et al. (2021) describe that the Fusarium solani strain 77-13-4 OE:fsr6 G418R by Nielsen M.R. et al. (2019) produces pigments such as aurofusarin, bikaverin and fusarubins under selected cultivation conditions. Nielsen M.R. et al. (2019) describe a vector system for targeted integration and overexpression of genes in Fusarium solani, wherein the Zn(II)₂Cys₆ transcriptional factor fsr6 controlling mycelial pigmentation was cloned and overexpressed. Thereby, Nielsen et al. (2019) targeted and activated the fusarubin (PKS3:fsr) gene cluster.

Menezes Bruna S. et al. (2020) describe pigment production by *Fusarium solani* BRM054066.

Molelekoa Tumisi Beiri J. et al. (2021) describe the production of pigments by filamentous fungi cultured of agro-industrial by-products using submerged and solid-state fermentation methods.

Rathna Janarthanam et al., (2016) describe the production of naphthoquinones and phenolics by *Fusarium solani* PSC-R of Palk Bay origin.

Venil CK et al. (2020) describe fungal pigments as potential coloring compounds for textile dyeing. However, the authors also point out that there is a necessity to explore novel pigments producing fungi in order to meet the existing demand for natural pigments. Thus, there is an urgent need for new biological and sustainable production systems for dyes and for biological and sustainable dyeing of substrates such as textiles.

### SUMMARY OF THE INVENTION

It is the objective of the present invention to provide a biological system for the sustainable production of dyes and pigments and for a sustainable colorization method of substrates.

The objective is solved by subject matter of the present invention.

It has been surprisingly shown that a specific isolated fungus belonging to the species *Fusarium solani* is able to produce a red dye which can be used for changing the color of different substrates. This isolated fungus lacks an artificial targeted activation of a gene cluster involved in dye production e.g., the isolated fungus of the invention lacks an artificial activation of the PKS3:fsr gene cluster. Furthermore, it has been surprisingly shown that the substrate can be colorized directly by incubating the substrate with the fungus or by incubating the isolated dye with the substrate. Even more surprisingly, it has been shown that the fungal dye is converted to a dark dye or pigment if FeCl₃ is added to the fungal dye and that said dark pigment can also be used for the colorization of a substrate.

According to the invention there is provided an isolated fungus belonging to the species *Fusarium solani* deposited under the number DSM 34187 at the Leibnitz Institute DSMZ-German Collection of Microorganisms and Cell Cultures GmbH on February 24, 2022.

According to the invention, said isolated *Fusarium solani* is specifically used for dyeing a substrate.

According to a further embodiment of the invention, a method for changing the color of a substrate is provided, comprising the sequential steps of:
i. providing an inoculum of the isolated fungus *Fusarium solani* deposited under the number DSM 34187;
ii. inoculating a cultivation medium with the inoculum;
iii. optionally pre-cultivating the inoculated cultivation medium and optionally inactivating the fungus after pre-cultivating;
iv. contacting the substrate with the cultivation medium of ii. or iii. until the desired color is obtained, and
v. heating the substrate.

According to a specific embodiment, the cultivation medium comprises a carbohydrate source, specifically glucose, preferably in the range of 1 to 4% (m/v).

Specifically, pre-cultivating in iii. and/or contacting in iv. is performed at a pH in the range of pH 4.8 to 6.7., specifically in the range of pH 5.0 to 6.5.

Specifically, pre-cultivating in iii. and/or contacting in iv. is performed at a temperature in the range of 15°C to 35°C, specifically in the range of 25°C to 28°C.

More specifically, pre-cultivating in iii. and/or contacting in iv. is performed under aerobic conditions.

Specifically, the method for changing the color of a substrate described herein further comprises contacting of the substrate with FeCl₃, wherein said contacting specifically leads to a decrease in lightness (L) according to the HSL color model.

According to a further embodiment of the invention, herein provided is a method for changing the color of a substrate comprising the method described herein, further comprising contacting of the substrate with FeCl₃. Specifically, contacting the substrate with FeCl₃ leads to a decrease in lightness (L) according to the HSL color model.

According to a further embodiment, herein provided is also a method for producing a fungal dye comprising the sequential steps of:
i. providing an inoculum of the isolated fungus *Fusarium solani* deposited under the number DSM 34187;
ii. inoculating a cultivation medium with the inoculum;
iii. cultivating the inoculated cultivation medium;
iv. harvesting the biomass and/or the cultivation medium; and
v. optionally extracting the dye from the harvested material of iv..

According to a specific embodiment, the cultivation medium comprises a carbohydrate source, preferably glucose, optionally further comprising peptone.

Specifically, cultivating in iii. is performed at a pH in the range of pH 5.0 ± 0.2 to 6.5 ± 0.2.

Specifically, cultivating in iii. is performed at a temperature in the range of 15°C to 35°C, preferably in the range of 25°C to 28°C.

In a specific embodiment, cultivating in iii. is performed under aerobic conditions.

More specifically, cultivating in iii. is performed at least until the development of a red/purple color is detected in the cultivation medium and/or in the biomass.

According to an embodiment, the cultivation medium is a liquid or a solid medium.

According to a specific embodiment, the method for producing a fungal dye described herein further comprises the addition of FeCl₃ for producing a dark pigment, specifically said pigment has a lightness (L) in the range of 0 to 20%.

Herein provided is also a method for extracting a fungal dye, said method comprising the sequential steps of:
i. drying harvested biomass and/or harvested cultivation medium comprising the fungus *Fusarium solani* deposited under the number DSM 34187;
ii. suspending the dried material from i. in a solvent, preferably in an alcohol, more preferably in ethanol;
iii. evaporating the solvent of ii.;
iv. resuspending the residuum of iii. in a solvent different from the solvent of ii., preferably in an ester, more preferably in ethyl acetate;
v. evaporating the solvent of iv.; and
vi. optionally resuspending the residuum of v..

According to the invention, the isolated *Fusarium solani* of the invention can be used to produce a dye or the dye can be extracted from said fungus as described herein. Said dye has a color ranging of RGB 170 ± 50, 10 ± 5, 39 ± 10, ranging from cherry red, dark red, brown red and Bordeaux red or any variations thereof.

Also described herein is a method for changing the color of a substrate comprising the sequential steps of:
i. contacting the substrate with the dye described herein until the desired color is obtained, and
ii. heating the substrate.

Specifically, heating the substrate is specifically performed at a temperature in the range of 60°C to 121°C, specifically performed for at least 20 minutes.

Provided herein is also a method for producing a dark pigment comprising the addition of FeCl₃ to the dye described herein or comprising the addition of FeCl₃ to the production method described above.

Further described herein is a dark pigment produced by the method described herein, wherein said pigment has a lightness (L) in the range of 0 to 20%.

According to a further embodiment, herein provided is also a method for changing the color of a substrate comprising the sequential steps of contacting the substrate with the pigment described herein until the desired color is obtained, and heating the substrate.

According to an embodiment, the substrate is a textile material selected from the group consisting of natural textile material, synthetic textile material, and combinations of natural and synthetic textile materials.

Specifically, the natural textile material can be, but is not limited to cotton, silk, wool, abacá, coir, linen, hemp, wood, cashmere, mohair.

Specifically, the synthetic textile material can be, but is not limited to polyester, rayon, acrylic, polycarbonate, polyethylene, spandex, acetate, lyocell, modal.

For dying the substrate, the substrate can be heated at a temperature in the range of 60°C to 121°C, specifically said heating lasts for at least 20 minutes.

According to yet a further embodiment, the dye produced by the inventive *Fusarium solani* deposited under the number DSM 34187 has antimicrobial activity.

Specifically encompassed herein is also a substrate having antimicrobial activity, wherein said substrate is dyed with the dye produced by the inventive *Fusarium solani* deposited under the number DSM 34187.

### FIGURES

**Figure 1****:** Phase contrast microscopy of FS IIa at 40 x magnification (A), 100 x magnification (B) and phase contrast microscopy of FS IIa at 40 x magnification showing a microconidium with 3 septae in comparison (C).
**Figure 2****:** Incubation of Fusarium solani on Sabouraud agar (A), Incubation of Fusarium solani in Sabouraud bouillon (side view: B, top view: C).
**Figure 3****:** Extraction with ethyl acetate results in a red dye/pigment (left flask). Addition of FeCl₃ triggers the formation of a dark and insoluble complex (right flask).

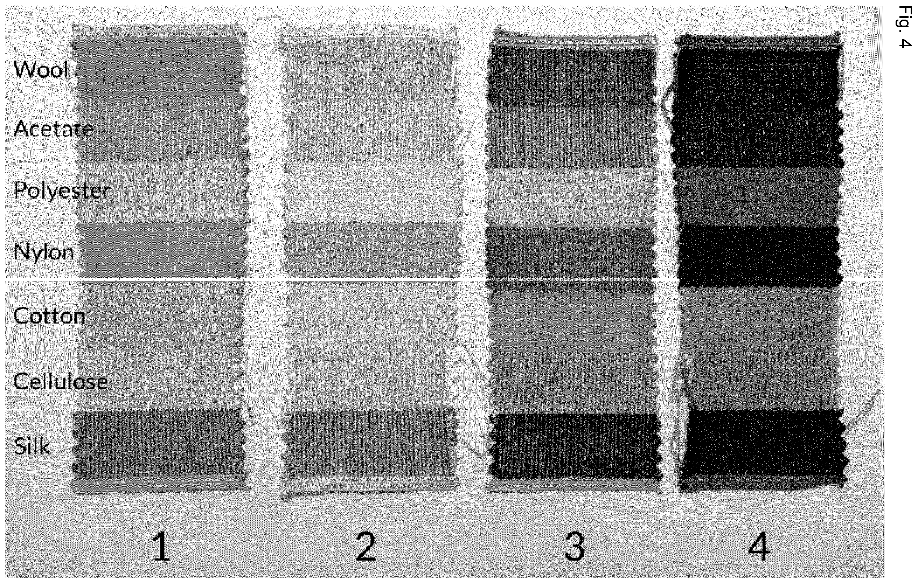

**Figure 4****:** Multifibres (MF) dyed with FS IIa. 1 - MF is incubated in a 72 hrs culture of FS IIa for 3 hrs at RT, followed by fixation of pigment at 60 °C for 25 minutes. 2 - Subsequent overnight incubation in FeCl₃ * 6 H2O (0.1 M, 0.03 g ml⁻¹) changes the colour via complex formation of pigment and FeCl₃. 3 - MF is incubated for 1.5 hours in a pigment dye bath followed by a heat treatment at 60 °C for 25 minutes. After cooling down, a second incubation in FeCl₃ solution results in a colour change to grey/black for cotton, cellulose and silk. 4 - MF is incubated for 1.5 hours in a highly concentrated pigment dye bath and heat treated at 60 °C for 25 minutes.
**Figure 5****:** Colour measurement according to RGB system
**Figure 6****:** Colour measurement according to HSL system

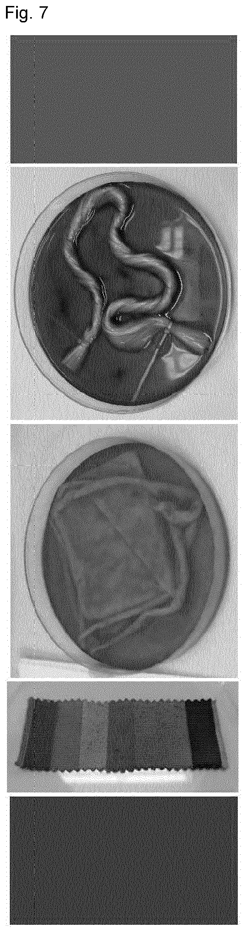

**Figure 7****:** Comparison of the red dye of the invention (three images in the middle) to commercial red dyes/colours (left image showing bordeaux colour and right image showing Barbados cherry colour located on the outer sides of the figure).
**Figure 8****:** Results of disc test on antimicrobial activity of the dye of the invention
**Figure 9****:** *N. lichenicola* IIa (DSM 34187, left) versus *N. solani* DSM 62805 (right). 4 days growth on Sabouraud agar at 28°C. Both strains produced a red-brownish pigment with isolate IIa showing a higher intensity in colour or higher amount of pigment. Both reached a diameter of ~ 4 cm.
**Figure 10****:** *N. lichenicola* IIa (DSM 34187, left) versus *N. solani* DSM 62805 (right). 45 days growth on Sabouraud agar at + 4°C. Aerial mycelium of isolate IIa shows increased pigmentation, however both released pigment into the agar layer.
**Figure 11****:** *N. lichenicola* IIa (DSM 34187, left) versus *N. solani* DSM 62805 (right). 48 hours growth in Sabouraud bouillon at 28°C. Both strains released red pigment into the surrounding medium but again isolate IIa shows a more intense redish colour.
**Figure 12****:** *N. lichenicola* IIa (DSM 34187, left) versus *N. solani* DSM 62805 (right). 72 hours growth in Sabouraud bouillon at 28°C. Both strains released red pigment into the surrounding medium but again isolate IIa shows a more intense redish colour.
**Figure 13****:** Phase contrast microscopy of *N. lichenicola* IIa (DSM 34187).
**Figure 14****:** Phase contrast microscopy of *N. solani* DSM 62805.
**Figure 15****:** Chromatogram of molecule identification

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017); Berg et al, "Stryer Biochemie" Springer Verlag, 2018; and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wildtype) products. Though there can be a certain degree of sequence identity to the native structure, it is well understood that the materials, methods and uses of the invention, *e.g.,* specifically referring to isolated nucleic acid sequences, amino acid sequences, expression constructs, transformed host cells and modified proteins and enzymes, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

According to the invention there is provided an isolated fungus belonging to the species *Fusarium solani,* wherein said isolated fungus can be applied for the production of a dye applicable in substrate colorization and also for the direct colorization of a substrate. Currently commercially used dyes for substrate colorization are chemically synthesized. Biological dyes and dyeing procedures have a lower environmental impact.

The isolated fungus of the invention is deposited under the number DSM 34187, and is also termed *Fusarium solani* IIa (FS IIa) or *Neocosmospora lichenicola* herein. Both terms can be used interchangeably.

Fungi belonging to the species *Fusarium solani* are filamentous fungi in the Ascomycota division. *Fusarium solani* is a common soil fungus belonging to the division of Ascomycetes. More than 60 species of this filamentous fungus are combined within the *Fusarium solani* Species Complex (FSSC) (Coleman, 2016). Within the *Fusarium solani* species complex (FSSC), many species are known and taxonomically, phylogenetically, and morphologically described (Kristensen et al., 2021). According to Short et al (2013) FSSC is a diverse complex of many phylogenetically distinct species. No obvious differences seem to occur in the morphology of divers *Fusarium solani* species (Schroers HJ et al. 2016; Chehri K et al, 2015; Matuo T and Snyder WC, 1972).

Surprisingly, the isolated *Fusarium solani* IIa of the invention produces a red dye in high yields and short time e.g., after overnight incubation. Using this dye or the isolated fungus, dyeing methods are enabled which allow direct penetration of the color and thus, the adsorption of the pigment/dye onto the textile fiber or material surface.

The term "changing the color" as used herein refers to a change in the color of a substrate. Thus, the visual appearance of the substrate changes.

Colors can be described using color models. The RGB color model is one method for describing a color. The RGB color model is an additive color model in which the red, green, and blue primary colors of light are added together in various ways to reproduce a broad array of colors. An alternative method for describing a color is the CYMK model or the HSL color model. HSL stands for hue, saturation, and lightness.

The term "substrate" as used herein refers to any material which can be a target of color changing. Thereby, the substrate may be a textile material but also a raw material which is converted into a textile material such yarns or threads.

The term "textile material" as used herein refers to a material which is made by creating an interlocking bundle of yarns or threads, which are produced by spinning raw fibers into long and twisted lengths. The raw fibers may be of natural or synthetic sources. Textile materials are formed by weaving, knitting, crocheting, knotting, tatting, felting, bonding, or braiding these yarns together. The terms "textile material", "textile", and "fabric" may be used interchangeably herein.

According to a specific embodiment, the substrate is a textile material selected from the group consisting of natural textile materials, synthetic textile materials, and combinations of natural and synthetic textile materials.

Natural textile materials can be, but are not limited to cotton, silk, wool, abacá, coir, linen, hemp, wood, cashmere, and mohair, or any combinations thereof.

Specifically, synthetic textile materials can be, but are not limited to polyester, rayon, acrylic, polycarbonate, polyethylene, spandex, acetate, lyocell, and modal, or any combinations thereof.

The terms "dye" and "pigment" are used herein to distinguish the red dye produced by the fungus of the invention from the dark pigment produced by adding FeCl₃ to the red dye. Thereby, the term "dye" is used for water-soluble red dye. The term "pigment" is used for the less water-soluble dark pigment.

According to one embodiment of the invention, a method for changing the color of a substrate is provided. Said method comprises the steps of providing an inoculum of the isolated fungus of the invention, inoculating a cultivation medium with the inoculum, optionally pre-cultivating the inoculated cultivation medium and optionally inactivating the fungus after pre-cultivating, contacting the substrate with the cultivation medium of one of the two previous steps until the desired color is obtained, and heating the substrate.

According to a specific embodiment, said method for changing the color of a substrate comprises the steps of providing an inoculum of the isolated fungus of the invention, inoculating a cultivation medium with the inoculum, contacting the substrate with the inoculated cultivation medium until the desired color is obtained, and heating the substrate.

According to a specific embodiment, said method for changing the color of a substrate comprises the steps of providing an inoculum of the isolated fungus of the invention, inoculating a cultivation medium with the inoculum, pre-cultivating the inoculated cultivation medium and optionally inactivating the fungus after pre-cultivating, contacting the substrate with the pre-cultivated cultivation medium until the desired color is obtained, and heating the substrate.

According to a specific embodiment, said method for changing the color of a substrate comprises the steps of providing an inoculum of the isolated fungus of the invention, inoculating a cultivation medium with the inoculum, pre-cultivating the inoculated cultivation medium and inactivating the fungus after pre-cultivating, contacting the substrate with the pre-cultivated cultivation medium until the desired color is obtained, and heating the substrate.

The term "inoculum" as used herein refers to a population of the fungus of the invention that is introduced in a cultivation medium or any suitable medium for growing the fungus.

According to a specific embodiment, the inoculum may be in solid or liquid form. The inoculum may be a fresh mycelium or a taken from a frozen culture collection such as in the form of a cryo culture. The inoculum may comprise a solid medium comprising a culture of the fungus of the invention e.g., the fungus grown on an agar plate. Said solid fungal inoculum may be pre-cultured for several days prior to inoculation. Specifically, said solid fungal inoculum may be pre-cultured for 1 to 30 days or even longer depending on the cultivation conditions of pre-culturing. Alternatively, the inoculum may comprise a liquid culture of the fungus of the invention. Said liquid inoculum may be pre-cultured for several days prior to inoculation. Specifically, said liquid inoculum may be pre-cultured for 1 to 30 days or even longer depending on the cultivation conditions of pre-culturing. Specifically, the concentration of the inoculum may vary depending on the form of the inoculum and on the pre-culturing of the inoculum.

The term "inoculating a cultivation medium" as used herein refers to the transfer of the inoculum into the cultivation medium.

According to a specific embodiment, the isolated fungus of the invention is cultivated in various methods described herein. The term "cultivation" and "pre-cultivating" is used herein for the growth under conditions outside of the natural environment of the fungus.

According to a specific embodiment, in the case the fungus was not inactivated prior to contacting the substrate with the cultivation medium, the fungus is cultivated also in the step of contacting. In other words, if the substrate is contacted with the cultivation medium comprising a viable fungus, the color of the substrate is changed simultaneously with the growth of the fungus. Thus, the fungal produced dye is directly used for changing the color of the substrate.

The term "contacting" as used herein refers to the step of bringing the substrate into contact with the dye or the fungus producing the dye. An example of contacting is the addition of the substrate directly into the liquid cultivation medium in which the fungus is cultivated or still present. Alternatively, if the isolated fungal dye is used for changing the color of the substrate, contacting is performed by adding the substrate into the solution of the dye.

According to a specific embodiment of the invention, inactivating the fungus may be performed by heating the fungal culture or the cultivation medium comprising the fungus to a temperature of about 121°C for at least 20 minutes.

According to a specific embodiment, the substrate is removed from the cultivation medium after the step of contacting and prior to the heating step.

According to a specific embodiment, the step of heating the substrate is applied to fix the color.

According to specific embodiment, heating the substrate is performed at a temperature in the range of 60°C to 121°C. Specifically, a temperature of 121°C is used if the heating step is also used for the inactivation of the fungus. Specifically, heating the substrate is performed at a temperature in the range of 60°C to 65°C, 65°C to 70°C, 70°C to 75°C, 75°C to 80°C, 80°C to 85°C, 85°C to 90°C, 90°C to 95°C, 95°C to 100°C, 100°C to 105°C, 105°C to 110°C, 110°C to 115°C, 115°C to 120°C, 100°C to 121°C, 115°C to 121°C, or at a temperature of 121°C. Specifically, heating the substrate is performed for at least 20 minutes up to several hours. Specifically, heating the substrate is performed for 20, 25, 30, 35, 40, 45, 50, 55, 60 minutes of even longer. Specifically, heating the substrate is performed for 20 to 25, 20 to 30, 20 to 40, 20 to 50, or 20 to 60 minutes. Specifically, heating may be performed by any method used in the field for heating a substrate. For example, the substrate may be heated in an oven or dryer.

According to one embodiment of the invention, the method for producing a fungal dye comprises the sequential steps of providing an inoculum of the isolated fungus of the invention, inoculating a cultivation medium with the inoculum, cultivating the inoculated cultivation medium, harvesting the biomass and/or the cultivation medium, and optionally extracting the dye from the harvested material.

According to a specific embodiment, the cultivation medium can be a liquid or a solid medium.

According to a specific embodiment, in the method for changing the color of a substrate using the fungus, the cultivation medium is a liquid medium.

According to a specific embodiment, in the method for producing the fungal dye, the cultivation medium can be a solid medium or a liquid medium.

In general, a solid cultivation medium comprises a substance for solidification of the cultivation medium such as agar but may comprise the same concentrations of other components as in the respective liquid medium.

According to a specific embodiment, the cultivation medium comprises a carbohydrate source.

According to a specific embodiment, the cultivation medium comprises glucose as carbohydrate source.

According to a specific embodiment, the cultivation medium comprises glucose in the range of 1% to 4% (m/v). Specifically, the cultivation medium comprises 1%, 2%, 3%, 4% (m/v) glucose.

According to a specific embodiment, the cultivation medium may comprise a different carbohydrate source than glucose or the combination of glucose with a different carbohydrate source. Specifically, the cultivation medium may comprise maltose instead of glucose, or a combination of maltose and glucose.

According to a specific embodiment, the cultivation medium comprises peptone.

According to a specific embodiment, the cultivation medium comprises 1%, 5%, 10%, 15%, 20%, 1 to 5%, 1 to 10%, 5 to 10%, 10 to 15%, 5 to 15%, or 1 to 15% (m/v) peptone.

According to a specific embodiment, the cultivation medium may comprise any other component commonly used in cultivation media. Non-limiting examples of such components are buffers, salts, yeast extract, malt extract, amino acids, starch, soybean meal, potato extract, rice extract, casein, dextrin, and antibacterial agents.

According to a specific embodiment, the cultivation medium is Sabouraud-Dextrose Agar.

According to a specific embodiment, the cultivation medium is Sabouraud-Dextrose bouillon.

According to a specific embodiment, the cultivation medium has a pH in the range of pH 5.0 ± 0.2 to 6.5 ± 0.2. Specifically, the pH of the cultivation medium is pH 5.0 ± 0.2, 5.1 ± 0.2, 5.2 ± 0.2, 5.3 ± 0.2, 5.4 ± 0.2, 5.5 ± 0.2, 5.6 ± 0.2, 5.7 ± 0.2, 5.8 ± 0.2, 5.9 ± 0.2, 6.0 ± 0.2, 6.1 ± 0.2, 6.2 ± 0.2, 6.3 ± 0.2, 6.4 ± 0.2, or 6.5 ± 0.2.

According to a specific embodiment, said pH of the cultivation medium is maintained during the pre-cultivation of the fungus, during the cultivation of the fungus, during contacting the substrate with the cultivation medium comprising the inoculated cultivation medium, and/or during contacting the substrate with the cultivation medium comprising the optionally pre-cultivated inoculated cultivation medium and comprising the optionally inactivated fungus. The pH can be maintained by any known method such as using a buffered cultivation medium and/or adding acids or bases. The pH may be controlled by a control unit of a bioreactor.

According to a specific embodiment, pre-cultivating and cultivating of the fungus is performed at a temperature in the range of 10°C to 35°C. Specifically, pre-cultivating and/or cultivating of the fungus is performed at a temperature in the range of 15°C to 35°C. Specifically, at a temperature in the range of 15°C to 30°C. Specifically, at a temperature in the range of 15°C to 28°C. Specifically, at a temperature in the range of 20°C to 28°C. Specifically, at a temperature in the range of 25°C to 28°C. Specifically, at a temperature of 15, 16, 17, 18, 19, 20, 21, 22, 23, 24, 25, 26, 27, 28, 29, 30, 31, 32, 33, 34, or 35°C.

According to a specific embodiment, contacting the substrate with the cultivation medium comprising the inoculated cultivation medium is performed at a temperature in the range of 10°C to 35°C. Specifically, at a temperature in the range of 15°C to 35°C. Specifically, at a temperature in the range of 15°C to 30°C. Specifically, at a temperature in the range of 15°C to 28°C. Specifically, at a temperature in the range of 20°C to 28°C. Specifically, at a temperature in the range of 25°C to 28°C. Specifically, at a temperature of 15, 16, 17, 18, 19, 20, 21, 22, 23, 24, 25, 26, 27, 28, 29, 30, 31, 32, 33, 34, or 35°C.

According to a specific embodiment, contacting the substrate with the cultivation medium comprising the optionally pre-cultivated inoculated cultivation medium and optionally inactivated fungus is performed at a temperature in the range of 10°C to 35°C. Specifically, at a temperature in the range of 15°C to 35°C. Specifically, at a temperature in the range of 15°C to 30°C. Specifically, at a temperature in the range of 15°C to 28°C. Specifically, at a temperature in the range of 20°C to 28°C. Specifically, at a temperature in the range of 25°C to 28°C. Specifically, at a temperature of 15, 16, 17, 18, 19, 20, 21, 22, 23, 24, 25, 26, 27, 28, 29, 30, 31, 32, 33, 34, or 35°C.

According to a specific embodiment, the steps of pre-cultivating, cultivating, and contacting are performed under aerobic conditions. Aerobic conditions are conditions in which free oxygen or dissolved oxygen is present.

According to a specific embodiment, harvesting of the biomass and/or the cultivation medium may be performed by separating the fungal biomass from the liquid cultivation medium by e.g., centrifugation and/or filtration.

According to a specific embodiment, harvesting may also be performed by removing the solid medium from its container e.g., from a petri dish. Thereby, the solid medium comprising the fungus may be cut into pieces.

According to a specific embodiment, the cultivation of the fungus, the pre-cultivation of the fungus, the contacting of the substrate with the cultivation medium comprising the inoculated cultivation medium, and/or the contacting of the substrate with the cultivation medium comprising the optionally pre-cultivated inoculated cultivation medium and optionally inactivated fungus may be performed as submerged fermentation in a bioreactor. The bioreactor may be equipped with various systems and sensors for control and automation such as e.g., an oxygen supply system, a pH control system, and/or a mixing system.

According to another specific embodiment, for the production of the dye, the fungus can be cultivated on a solid medium such as via solid state fermentation. Thereby, the dye accumulates in the medium and/or in the mycelium of the fungus.

According to a specific embodiment, in the method for the producing a fungal dye, the step of cultivating is performed at least until the development of a red/purple color is detected in the cultivation medium and/or at the biomass. Thereby, the cultivation is performed until the desired intensity of color is produced by the fungus. The duration of this step may vary depending on the specific conditions of the cultivating step, also depending on the form and concentration of the used inoculum of the fungus.

According to another embodiment, the dye can be extracted from the harvested biomass and/or from the cultivation medium. In the extraction method of the invention, the final residuum may be resuspended. The final residuum may also be further processed for storage of the dye e.g., by production of a powder.

According to one embodiment, the color of the dye produced by the fungus of the invention is in the range of RGB 170 ± 50, 10 ± 5, 39 ± 10 using the RGB color model.

According to one embodiment, the lightness (L) of the dye produced by the fungus is in the range of 25-45% according to the HSL color model.

According to a specific embodiment, *Fusarium solani* IIa produces a red pigment whose color darkens with prolonged growth to dark-red, brown-red and bordeaux red.

According to one embodiment, the fungal dye can be used for changing the color of a substrate. Specifically, the color of a substrate can be changed by the sequential steps of contacting a substrate with the fungal dye until the desired color is obtained, and heating the substrate. As described previously herein, the heating step is performed to fix the color and is performed as describes elsewhere herein. The resulting color of the textile depends on the duration of the contacting step and also on the concentration of the dye.

According to a specific embodiment, in the step of contacting the substrate with the fungal dye, the dye may be present in liquid or solid form. Specifically, the substrate may be dipped into a solution comprising the dye. More specifically, the substrate may be fully dipped into the solution comprising the dye or may be partly dipped into the said solution. Alternatively, a solution of the dye or the dye in powder form may be sprinkled over the substrate. The substrate may be pre-treated or pre-wetted before the application of the dye. The solution of the dye may be sprayed onto the substrate. Different additives may be added to the solution of the dye e.g., binders and/or thickeners may be added to the solution of the dye for the formation of a paste.

According to another embodiment , a dark pigment is produced by addition of FeCl₃ to the dye of the invention. Specifically, said dark pigment has a lightness (L) in the range of 0 to 20%. More specifically, said dark pigment has a lightness (L) of 0, 1, 2, 3, 4, 5, 6, 7, 8, 9, 10, 11, 12, 13, 14, 15, 16, 17, 18, 19, or 20%.

According to a specific embodiment, the lightness (L) of the dark pigment is reduced compared to the lightness (L) of the fungal red dye.

According to another embodiment, the color of a substrate previously dyed with the fungus of the invention as described herein or with the fungal dye as described can be further changed by addition of FeCl₃ to the dyed substrate. Thereby, the color of the substrate changes towards a darker color. The resulting color of said further treated substrate may be dependent on the color intensity of the prior dyeing step and may also be dependent on the material of the substrate.

According to a specific embodiment, the lightness (L) of the color of the substrate is decreased compared to the color of the substrate prior to contacting with FeCl₃. In other words, the contacting of a dyed substrate of the invention with FeCl₃ leads to a decrease in the lightness (L) according to the HSL color model. Said decrease in lightness (L) compared to the L-value prior to the application of FeCl₃ may be by 10, 20, 30, 40, 50%, or even more.

According to a specific embodiment, the color of a substrate can be changed by contacting the substrate with FeCl₃, wherein said substrate was dyed by the fungus or by the fungal dye of the invention prior to contacting with FeCl₃.

According to a specific embodiment, contacting of a substrate pre-dyed with the dye of the invention with FeCl₃ leads to the formation of a dark color.

According to another embodiment, the color of a substrate can be changed by a method comprising the sequential steps of contacting the substrate with the dark pigment of the invention until the desired color is obtained, and heating the substrate. Again, heating is performed to fix the color.

According to another embodiment, the fungal dye of the invention has antimicrobial activity.

The term "antimicrobial activity" refers to all active principles that inhibit the growth of bacteria, prevent the formation of microbial colonies, and may destroy microorganisms.

According to another embodiment, the substrate dyed with the dye of the invention has antimicrobial activity. Specifically, the growth of microorganisms on said substrate is inhibited.

According to a specific embodiment, a textile material dyed with the dye of the invention has antimicrobial activity. Specifically, the growth of microorganisms on or in close proximity to the textile material is inhibited.

The examples described herein are illustrative of the present invention and are not intended to be limitations thereon. Many modifications and variations may be made to the techniques described and illustrated herein without departing from scope of the invention. Accordingly, it should be understood that the examples are illustrative only and are not limiting upon the scope of the invention.

### EXAMPLES

### Example 1: Cultivation of Fusarium solani of the invention (FS IIa)

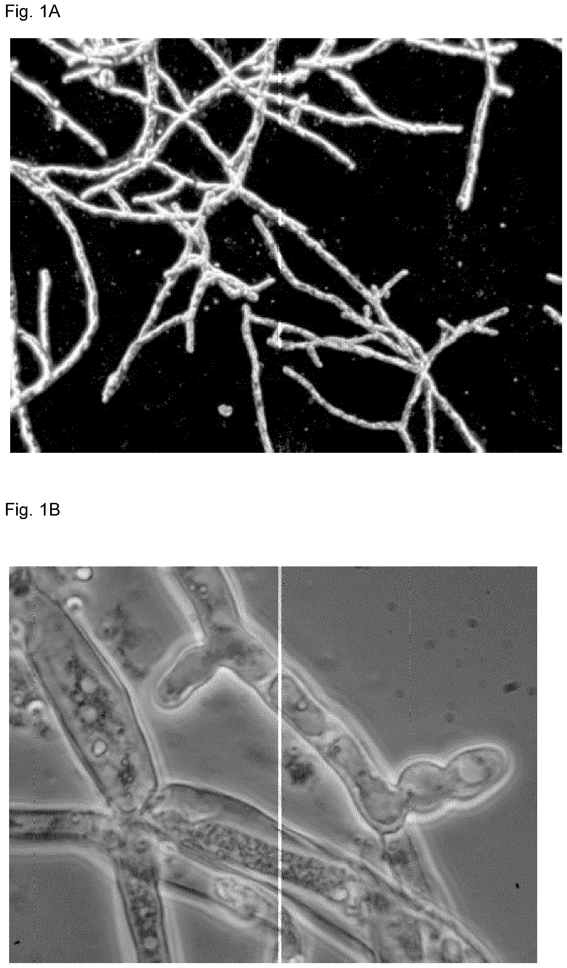

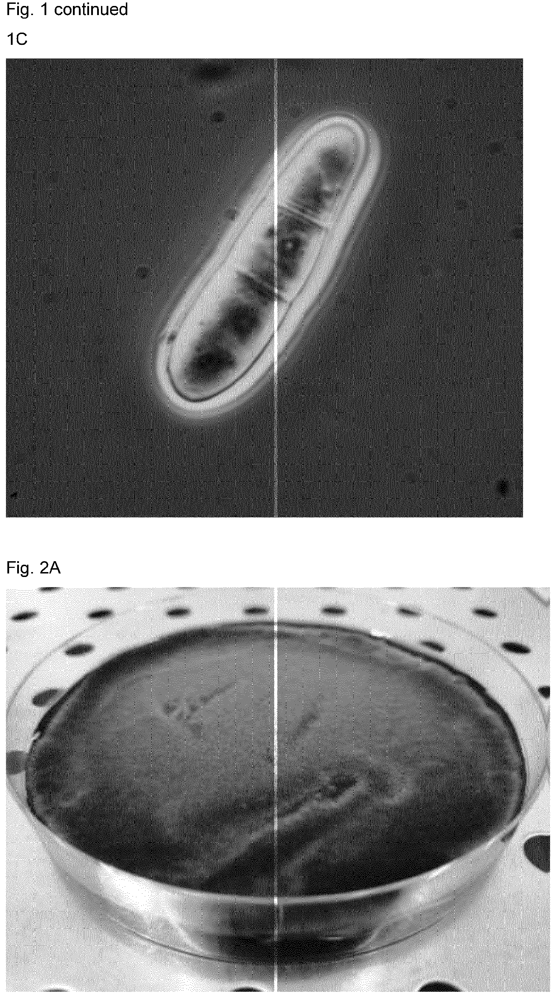

Cultivations of *Fusarium solani* (FS) IIa were done in Luria-Bertani, Sabouraud and wort broth, respectively. Microscopy reveals hyphal growth. The body is formed by filaments or hyphae (Figure 1 A, 1 B, 1 C), which follows typical mycelial growth pattern of fungi belonging to the phylum Ascomycetes.

FS IIa is cultivated in a temperature range from 25°C to 28°C aerobically.

### Example 2: Production of dye

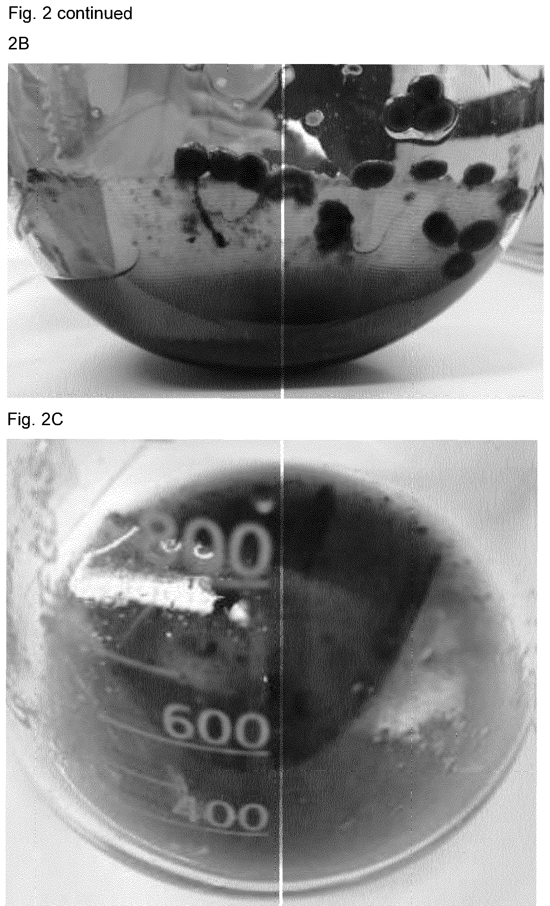

Fusarium solani IIa is grown on Sabouraud 4 % Glucose agar (Carl Roth, X932.2) as well as in Sabouraud 2 % Glucose Bouillon (Carl Roth, AE23.1) at 28 °C. Using this complex medium, a dark red-brown dye (see Figures 2A, 2B and 2C) is formed after overnight incubation (~12 to 16 hours), which diffuses into the agar or into the surrounding medium and colors the mycelium red. In liquid medium, dye formation can take up to 48 hours.

Colonies of FS IIa appear Bordeaux red to dark violet on Sabouraud dextrose agar containing 40 g/l dextrose and 10 g/l peptone at a pH of 5.6. Colonies develop pigmentation after 24 hours of incubation at 25°C to 28°C. The pigment diffuses into the agar leading to a dark Bordeaux red colored agar - almost appearing black.

Cultivation in Sabouraud dextrose broth containing 20 g l⁻¹ dextrose and 10 g l⁻¹ peptone at a pH of 5.6 results in a Bordeaux red colored medium and Bordeaux red colored rounded up mycelium after 24 hours incubation at 28°C at 150 rpm in a baffled or non-baffled Erlenmeyer flask.

Figure 2A shows Fusarium solani incubated overnight on Sabouraud agar, Figures 2B and 2C show Fusarium solani incubated overnight in Sabouraud bouillon.

### Example 3: Dye

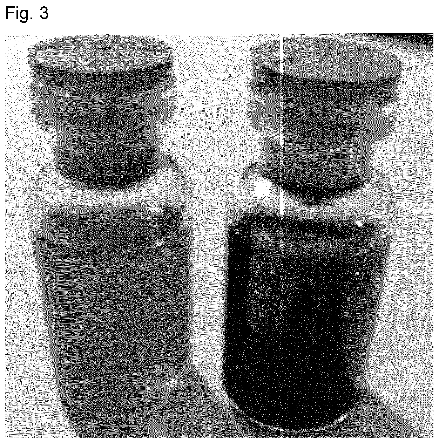

Extraction of the dye with ethyl acetate results in a red dye (see Figure 3, left flask). Addition of FeCl₃ triggers the formation of a dark and insoluble complex (see Figure 3, right flask).

### Example 4: Extraction of dye

Extraction of this Bordeaux red pigment was done according to the following protocol:
- After an incubation period of 10 days, the colored agar/medium including the colored mycelium of FS II on the surface is reduced to small pieces and dried.
- Dried pieces are suspended in EtOH (+10 % dH2O) and heated up to approx. 80°C for 20 minutes. Alternatively, evaporation can be done at room temperature over a period of 3 days.
- The residuum is resuspended in dH₂O, and a first extraction is done via addition of ethyl acetate in a separation funnel. After evaporation of ethyl acetate, a sticky, intensively red matter remains.
- This red matter is resuspendable in dH₂O, ethyl acetate, acetic acid and ethanol.

### Example 5: Dyeing

3 ml of fresh medium are inoculated with fresh mycelium or directly from a cryo culture with an inoculation loop. After overnight incubation at 28°C (stirred), fresh 200 ml medium are inoculated with the starter culture from the day before.

Blank textile samples are added immediately at cultivation start or are added after sufficient pigment formation. Depending on that, the dyeing time frame ranges from 2 hours to 72 hours resulting in different color intensity.

Blank textile samples are incubated in a dye bath containing resuspended red pigment. The colorization starts immediately and intensity increases with time.

Each dyeing is finalized with a heat treatment from 60°C to 90°C for 30 minutes which inactivates the fungus (up to 121°C) and fixates the pigment on to material fibers.

### Example 6: Dyeing

The fungus is cultured in liquid complex medium. After a pre-culture, the cells are inoculated in fresh liquid medium and grow in the presence of the fabric to be dyed. The dyeing process is stopped as soon as color intensity is reached. Subsequent heat treatment (60°C for 20 minutes or 90°C for 20 minutes) fixates the pigment within the textile.

### Example 7: Dyeing

Multifibers (MF) were incubated in a 72 hrs culture of FS IIa for 3 hrs at RT, followed by fixation of pigment at 60 °C for 25 minutes. The results of the dyeing process are shown in row 1 of Figure 4.

Subsequent overnight incubation of the dyed substrate in FeCl₃ * 6 H2O (0.1 M, 0.03 g ml-1) changes the colour via complex formation of pigment and FeCl₃ (see row 2 of Figure 4).

### Example 8: Dyeing

The fungus is grown until reaching a desired density as well as dye production, afterwards it is inactivated using heat (121 °C for 20 minutes). Only following inactivation of the fungus, the fabric is added to the culture and incubated until a desired color is reached. Heat treatment finalizes the dyeing procedure.

### Example 9: Dyeing

MF were incubated for 1.5 hour in a pigment dye bath followed by a heat treatment at 60°C for 25 minutes. After cooling down, a second incubation in FeCl₃ solution resulted in a colour change to grey/black for cotton, cellulose and silk. The results are shown in row 3 of Figure 4.

### Example 10: Dyeing

MF were incubated for 1.5 hours in a highly concentrated pigment dye bath and heat treated at 60°C for 25 minutes. The results are shown in row 4 of Figure 4.

### Example 11: Dyeing

Crude extract - partly soluble in H₂O - is used for dyeing. Incubation at room temperature for approx. 3 hours results already in a dark red colorization of textile/material. Heat treatment at 90°C for 30 minutes increases intensity and saturation of the color.

### Example 12: Dyeing

Additional to described dyeing procedures, the red dye of FS IIa forms a complex with FeCl₃ resulting in a color change to a dark color which is almost black for coloring silk.

### Example 13: Characterization of dyed substrates

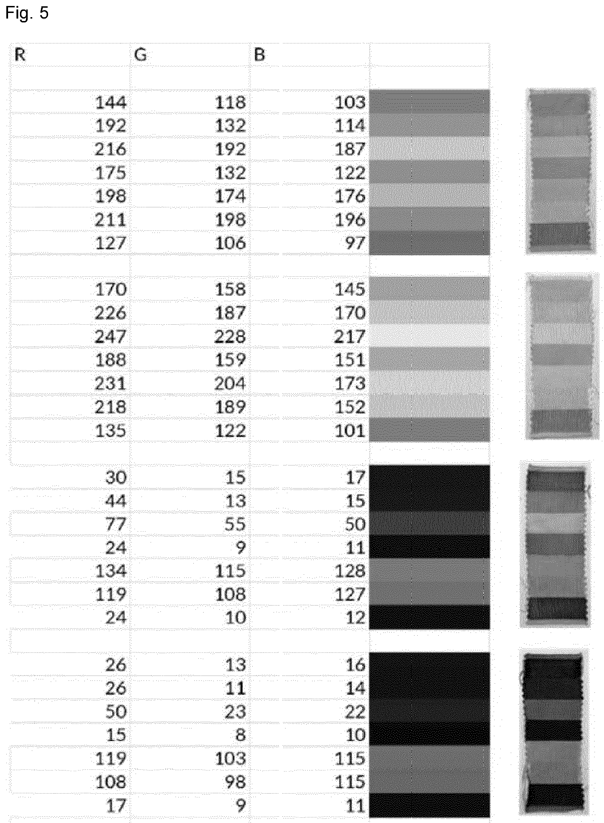

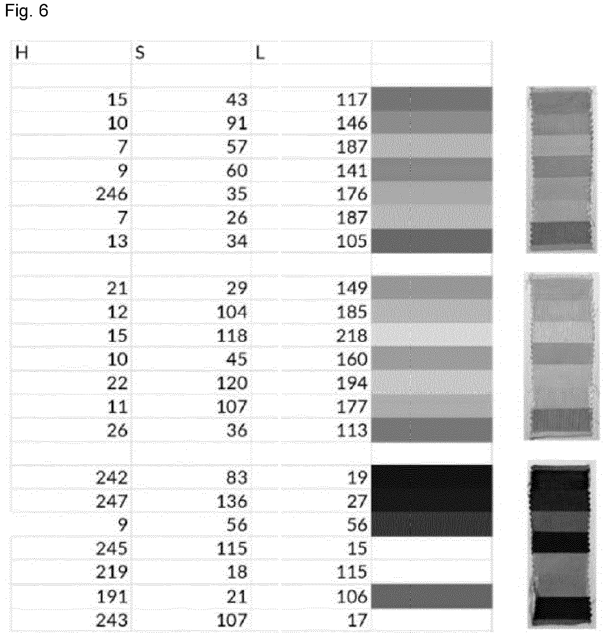

The dyed substrates shown in Figure 4 have been characterized using a color analysis device (RGB-2000, Voltcraft). The color was analysed for the RGB color model (see Figure 5) and for the HSL color model (see Figure 6).

### Example 14: Antimicrobial activity

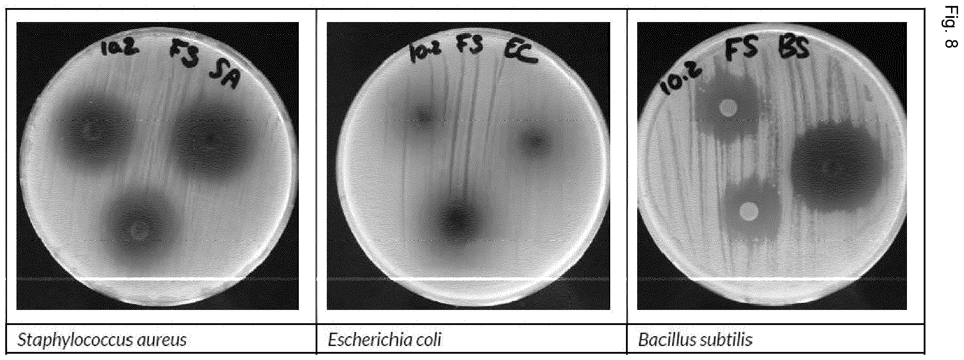

Antimicrobial activity was tested using a disc test. Thereby, the antimicrobial activity was tested against *Escherichia coli, Staphylococcus aureus, Pseudomonas sp.* and *Bacillus subtilis. Fusarium solani* shows antimicrobial activity against *Staphylococcus aureus* and *Bacillus subtilis* as well as against *Escherichia coli* to a weaker extent. The disc test was performed by incubating *Staphylococcus aureus, Escherichia coli,* and *Bacillus subtilis* at 37°C, for 18 hours on Müller-Hinton agar in the presence of discs prepared with 20 µL of the aqueous extract of the dye of the invention. The result of the antimicrobial activity is shown in Figure 8. The sensitivity of selected microorganisms to the extract of *Fusarium solani* IIa was determined via measuring the diameter of zones of inhibition (ZOI) in mm. The results of the antimicrobial activity are also shown in the following Table 1.

**Table 1**

| Microorganisms | ZOI (mm) |
|---|---|
| *Staphylococcus aureus* | 16-16-21 |
| *Escherichia coli* | 8-8-8 |
| *Bacillus subtilis* | 15-15-28 |

### Example 15: Sequencing/Re-identification of DSM 34187

After DNA extraction, partial gene tef (translocation elongation factor alpha-1) was sequenced using specific primers for the identification of *Fusarium sp.* Assembled DNA sequences were loaded at GenBank, MycoID and Fusarium ID database.

### Date of Sequencing: 31.03.2022

Sequencing by: **DSMZ,** Braunschweig (GER)
>ID 22-85 Tef

TCGACCTGGCAGTCGACCACCGTAAGTCAAACCTCATCGCGATCTGCTTATCTCG
GGTCGTGGAACCCCGCCTGGGCTCAGGCGGGGTATTCATCAGTCATTTCATGCT
GACAATCATCTACAGACCGGTCACTTGATCTACCAGTGCGGTGGTATCGACAAGC
GAACCATCGAGAAGTTCGAGAAGGTTGGTGACATCTCCCCGATCGCGCCTTGCTA
TTCCGCATCGAACTCCCCGTCGAATTCCCTCCCTCGCGATACGCTCTGCGCACCC
TTCTCCCGAGTCCCAAAAATTTTTGCGGTTCAGCCGTAATTTTCTCTTGGTGGGGT
TATCTACCCCGCCACTCGGGCGACGTTGGGCAAAGCCCTGATCCCTGCACACAA
AAACACCAAACCCTCTTGGCGCGCATCATCACGTGGTCCACGACAGACACTAACC
GATTCAACAATAGGAAGCCGCTGAGCTCGGTAAGGGTTCCTTCAAGTACGCCTGG
GTCCTTGACAAGCTCAAGGCCGAGCGTGAGCGTGGTATCACCATCGACATTGCT
CTCTGGAAGTTCGAGACTCCCCGCTACTATGTCACCGTCATTGGTATGTCGCTGT
CGTCTCTCAAACATGTCTCACCACTAACCATCAACAGACGCCCCCGGCCACCGTG
ACTTCATCAAGAACATGATCACTGG (SEQ ID NO:1)

### Sequencing result:

| Isolate | Species | GenBank Reference | Strain | Length/ Basepairs | Sequence similarity (%) |
|---|---|---|---|---|---|
| lia | *Fusarium lichenicola* | Tef LR583620.1 | CBS 623.92 Ex-Typus | 630/630 | 100 |

### Identification:

### Neocosmospora lichenicola (C. Massal.) Sand.-Den. & Crous, [MB#822901]

*Neocosmospora lichenicola* belongs to the *Fusarium solani* species complex, FSSC. *N. lichenicola* belongs to FSSC group 16b.

### Example 16: Morphology of DSM 34187

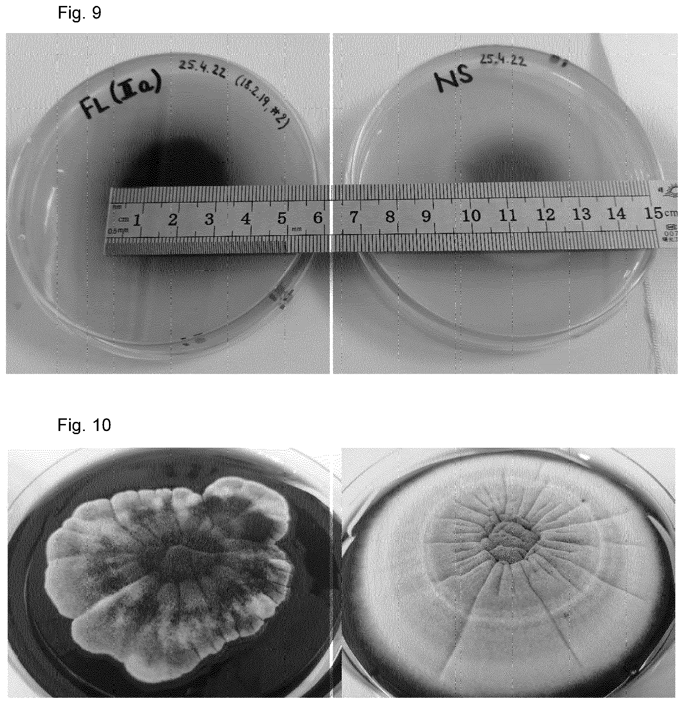

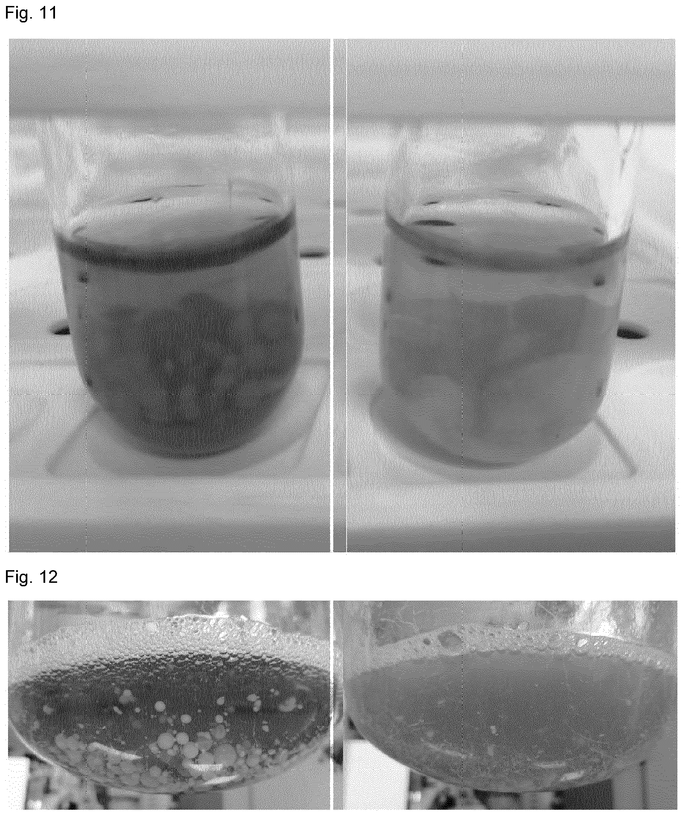

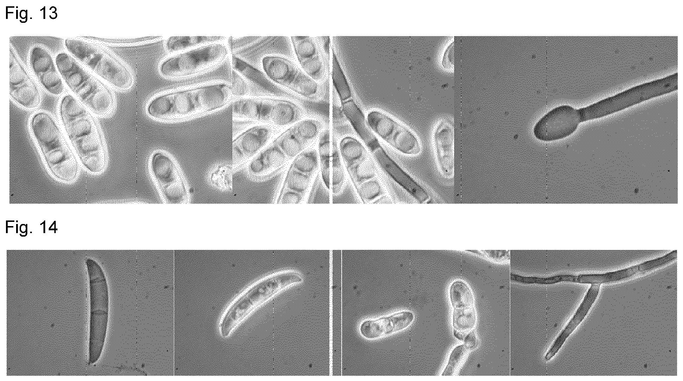

Strain DSM 62805 *Neocosmospora solani* (see reference L. Lombard, N.A. van der Merwe, J.Z. Groenewald, and P.W. Crous Studies in Mycology 80: 189-245) was used as a reference strain to the isolate IIa. First investigations of potential morphological differences were performed via growth and aerial mycelium observation as well as growth patterns in liquid culture. Both strains were cultivated on Sabouraud agar (4 % glucose) and Sabouraud bouillon (2 % glucose) at 28 °C. See Fig. 9 to Fig. 14.

### Example 17: Identification of molecule

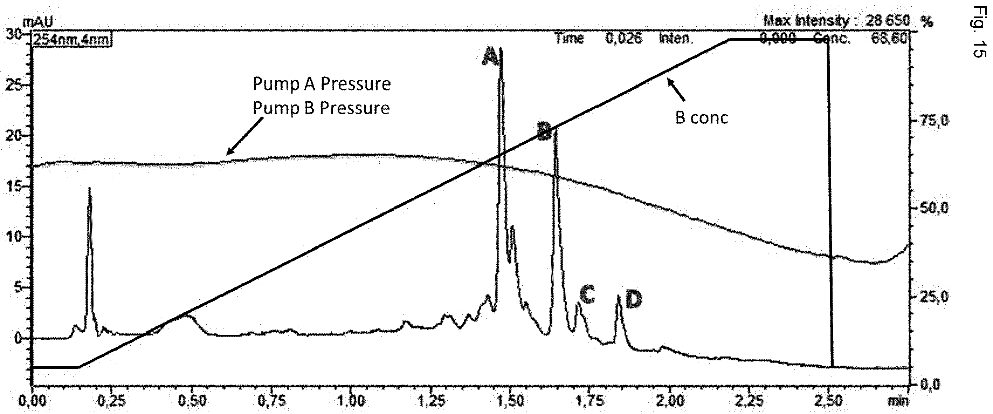

The crude extract of the red pigment - produced by isolate IIa - was dissolved in EtOH + 25% H₂O at a concentration of 0.5 mg ml⁻¹ and loaded into an HPLC column (see chromatogram in Fig. 15). The chromatogram shows peak A at a retention time of around 1.45-1.50 min at a mAU of around 29, peak B at a retention time of around 1.65 min at a mAU of around 21, peak C at a retention time of around 1.70 min at a mAU of around 3, and peak D at a retention time of around 1.85 min at a mAU of around 4.

### Example 18: Identification of molecule

The substances identified from the extract of *Fusarium lichenicola* IIa (DSM 34187) are shown in the following table 2:

**Table 2**

| | |
|---|---|
| MARTICIN (CAS #: 19196-45-3): 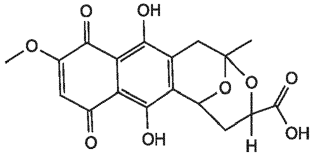 | Iso-MARTICIN: 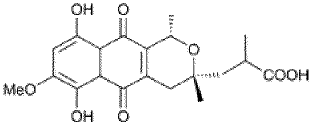 |
| BOSTRYCOIDIN (CAS #: 4589-33-7): 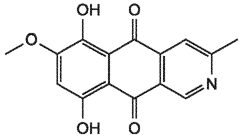 | ANHYDROFUSARUBIN (CAS #: 79383-28-1): 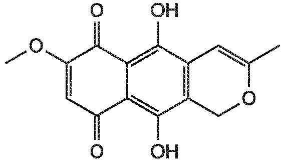 |

### REFERENCES

Chehri Khosrow, Salleh Baharuddin, Zakaria Latiffah (2014) Morphological and Phylogenetic Analysis of Fusarium solani Species Complex in Malaysia, Microb Ecol (2015) 69:457-471, DOI 10.1007/s00248-014-0494-2
Coleman JJ. The Fusarium solani species complex: ubiquitous pathogens of agricultural importance. Mol Plant Pathol. 2016;17(2):146-158. doi:10.1111/mpp.12289
Kristensen SB, Pedersen TB, Nielsen MR, Wimmer R, Muff J, Sørensen JL. Production and Selectivity of Key Fusarubins from Fusarium solani due to Media Composition. Toxins. 2021; 13(6):376. https://doi.org/10.3390/toxins13060376
Matuo Takken, Snyder William C. (1972) Use of Morphology and Mating Populations in the Identification of Formes Speciales in Fusarium solani, Phytopathology 63:562-565
Menezes Bruna S. et al., Pigment production by Fusarium solani BRM054066 and determination of antioxidant and anti-inflammatory properties. AMB Express, 2020, 10(1), XP93014625
Molelekoa Tumisi Beiri J. et al., Production of Pigments by Filamentous Fungi Cultured on Agro-Industrial by-Products Using Submerged and Solid-State Fermentation Methods. Fermentation, 2021, 7(4), 295
Nielsen, M.R., Holzwarth, A.K.R., Brew, E. et al. A new vector system for targeted integration and overexpression of genes in the crop pathogen Fusarium solani. Fungal Biol Biotechnol 6, 25 (2019). https://doi.org/10.1186/s40694-019-0089-2
Short DP, O'Donnell K, Thrane U, et al. Phylogenetic relationships among members of the Fusarium solani species complex in human infections and the descriptions of F. keratoplasticum sp. nov. and F. petroliphilum stat. nov. Fungal Genet Biol. 2013;53:59-70. doi:10.1016/j.fgb.2013.01.004
Schroers Hans-Josef, Samuels Gary J., Zhang Ning, Short Dylan P.G., Juba Jean & Geiser David M. (2016) Epitypification of Fusisporium (Fusarium) solani and its assignment to a common phylogenetic species in the Fusariumsolani species complex, Mycologia, 108:4, 806-819, DOI: 10.3852/15-255
Slama HB, Chenari Bouket A, Pourhassan Z, Alenezi FN, Silini A, Cherif-Silini H, Oszako T, Luptakova L, Golińska P, Belbahri L. Diversity of Synthetic Dyes from Textile Industries, Discharge Impacts and Treatment Methods. Applied Sciences. 2021; 11(14):6255. https://doi.org/10.3390/app11146255
Rathna Janarthanam et al., Production of naphthoquinones and phenolics by a novel isolate Fusarium solani PSC-R of Palk Bay and their industrial applications. Bioresource Technology, 2016, 213, 289-298
Venil CK, Velmurugan P, Dufossé L, Devi PR, Ravi AV. Fungal Pigments: Potential Coloring Compounds for Wide Ranging Applications in Textile Dyeing. J Fungi (Basel). 2020;6(2):68. Published 2020 May 20. doi:10.3390/jof6020068

## Claims

1. An isolated fungus belonging to the species *Fusarium solani* deposited under the number DSM 34187.

2. Use of the isolated fungus of claim 1 for dyeing a substrate.

3. The use of claim 2, wherein the substrate is a textile material selected from the group consisting of natural textile material, synthetic textile material, and combinations of natural and synthetic textile materials, specifically wherein the natural textile material is selected from the group consisting of cotton, silk, wool, abacá, coir, linen, hemp, wood, cashmere, and mohair, and wherein the synthetic textile material is selected from the group consisting of polyester, rayon, acrylic, polycarbonate, polyethylene, spandex, acetate, lyocell, and modal.

4. A method for changing the color of a substrate comprising the sequential steps of:
i. providing an inoculum of the isolated fungus of claim 1;
ii. inoculating a cultivation medium with the inoculum;
iii. optionally pre-cultivating the inoculated cultivation medium and optionally inactivating the fungus after pre-cultivating;
iv. contacting the substrate with the cultivation medium of ii. or iii. until the desired color is obtained, and
v. heating the substrate, specifically wherein heating the substrate is performed at a temperature in the range of 60°C to 121°C, specifically performed for at least 20 minutes.

5. The method of claim 4, wherein pre-cultivating in iii. and/or contacting in iv. is performed at a pH in the range of pH 4.8 to 6.7, specifically at a temperature in the range of 15°C to 35°C, specifically under aerobic conditions.

6. The method of claim 4 or 5, wherein the substrate is a textile material selected from the group consisting of natural textile material, synthetic textile material, and combinations of natural and synthetic textile materials, specifically wherein the natural textile material is selected from the group consisting of cotton, silk, wool, Abacá, coir, linen, hemp, wood, cashmere, and mohair, and wherein the synthetic textile material is selected from the group consisting of polyester, rayon, acrylic, polycarbonate, polyethylene, spandex, acetate, lyocell, and modal.

7. The method of any one of claims 4 to 6, wherein the cultivation medium comprises a carbohydrate source, specifically glucose, preferably in the range of 1 to 4% (m/v).

8. The method of any one of claims 4 to 7, wherein the cultivation medium is a liquid or a solid medium.

9. The method of any one of claims 4 to 8, further comprising contacting of the substrate with FeCl₃, wherein said contacting specifically leads to a decrease in lightness (L) according to the HSL color model.

10. A method for producing a fungal dye comprising the sequential steps of:
i. providing an inoculum of the isolated fungus of claim 1;
ii. inoculating a cultivation medium with the inoculum;
iii. cultivating the inoculated cultivation medium;
iv. harvesting the biomass and/or the cultivation medium; and
v. optionally extracting the dye from the harvested material of iv..

11. The method of claim 10, wherein the cultivation medium comprises a carbohydrate source, specifically glucose, preferably in the range of 1 to 4% (m/v).

12. The method of claim 10 or 11, wherein cultivating in iii. is performed at a pH in the range of pH 4.8 to 6.7, specifically at a temperature in the range of 15°C to 35°C, preferably in the range of 25°C to 28°C, specifically under aerobic conditions, specifically cultivating in iii. is performed at least until the development of a red/purple color is detected in the cultivation medium and/or in the biomass.

13. The method of any one of claims 10 to 12, wherein the cultivation medium is a liquid or a solid medium.

14. The method of any one of claims 10 to 13, further comprising the addition of FeCl₃ for producing a dark pigment, specifically said pigment has a lightness (L) in the range of 0 to 20%.

15. A method for extracting a fungal dye, said method comprising the sequential steps of:
i. drying harvested biomass and/or harvested cultivation medium comprising the fungus of claim 1;
ii. suspending the dried material from i. in a solvent, preferably in an alcohol, more preferably in ethanol;
iii. evaporating the solvent of ii.;
iv. resuspending the residuum of iii. in a solvent different from the solvent of ii., preferably in an ester, more preferably in ethyl acetate;
v. evaporating the solvent of iv.; and
vi. optionally resuspending the residuum of v..

## Patentansprüche

1. Isolierter Pilz, welcher der Spezies *Fusarium solani* zugehörig ist, hinterlegt unter der Kennzahl DSM 34187.

2. Verwendung des isolierten Pilzes nach Anspruch 1 zum Färben eines Substrats.

3. Verwendung nach Anspruch 2, wobei das Substrat ein textiles Material ausgewählt aus der Gruppe bestehend aus natürlichem textilem Material, synthetischem textilem Material und Kombinationen aus natürlichen und synthetischen textilen Materialien ist, wobei im Speziellen das natürliche textile Material ausgewählt ist aus der Gruppe bestehend aus Baumwolle, Seide, Wolle, Abaca, Kokosfaser, Leinen, Hanf, Holz, Kaschmir und Mohair, und wobei das synthetische textile Material ausgewählt ist aus der Gruppe bestehend aus Polyester, Rayon, Acryl, Polycarbonat, Polyethylen, Spandex, Acetat, Lyocell und Modal.

4. Verfahren zum Verändern der Farbe eines Substrats, umfassend die aufeinanderfolgenden Schritte:
i. Bereitstellen eines Inokulats des isolierten Pilzes nach Anspruch 1;
ii. Inokulieren eines Kulturmediums mit dem Inokulat;
iii. optional Vorkultivieren des inokulierten Kulturmediums und gegebenenfalls Inaktivieren des Pilzes nach dem Vorkultivieren;
iv. Inkontaktbringen des Substrats mit dem Kulturmedium aus ii. oder iii., bis die gewünschte Farbe erzielt wird, und
v. Erwärmen des Substrats, wobei im Speziellen das Erwärmen des Substrats bei einer Temperatur in dem Bereich von 60 °C bis 121 °C durchgeführt wird, und im Speziellen für zumindest 20 Minuten durchgeführt wird.

5. Verfahren nach Anspruch 4, wobei das Vorkultivieren in iii. und/oder das Inkontaktbringen in iv. bei einem pH-Wert in dem Bereich von pH 4,8 bis 6,7 durchgeführt wird, im Speziellen bei einer Temperatur in dem Bereich von 15 °C bis 35 °C, im Speziellen unter aeroben Bedingungen.

6. Verfahren nach Anspruch 4 oder 5, wobei das Substrat ein textiles Material ausgewählt aus der Gruppe bestehend aus natürlichem textilem Material, synthetischem textilem Material und Kombinationen aus natürlichen und synthetischen textilen Materialien ist, wobei im Speziellen das natürliche textile Material ausgewählt ist aus der Gruppe bestehend aus Baumwolle, Seide, Wolle, Abaca, Kokosfaser, Leinen, Hanf, Holz, Kaschmir und Mohair, und wobei das synthetische textile Material ausgewählt ist aus der Gruppe bestehend aus Polyester, Rayon, Acryl, Polycarbonat, Polyethylen, Spandex, Acetat, Lyocell und Modal.

7. Verfahren nach einem der Ansprüche 4 bis 6, wobei das Kulturmedium eine Kohlehydratquelle, im Speziellen Glucose, vorzugsweise in dem Bereich von 1 bis 4 % (m/v) umfasst.

8. Verfahren nach einem der Ansprüche 4 bis 7, wobei das Kulturmedium ein flüssiges oder ein festes Medium ist.

9. Verfahren nach einem der Ansprüche 4 bis 8, ferner umfassend das Inkontaktbringen des Substrats mit FeCl₃, wobei das Inkontaktbringen im Speziellen zu einer Verringerung der Helligkeit (L) gemäß dem HSL-Farbmodell führt.

10. Verfahren zur Herstellung eines Farbstoffs auf Pilzbasis, umfassend die aufeinanderfolgenden Schritte:
i. Bereitstellen eines Inokulats des isolierten Pilzes nach Anspruch 1;
ii. Inokulieren eines Kulturmediums mit dem Inokulat;
iii. Kultivieren des inokulierten Kulturmediums;
iv. Ernten der Biomasse und/oder des Kulturmediums; und
v. optional Extrahieren des Farbstoffs aus dem geernteten Material aus iv.

11. Verfahren nach Anspruch 10, wobei das Kulturmedium eine Kohlehydratquelle, im Speziellen Glucose, vorzugsweise in dem Bereich von 1 bis 4 % (m/v) umfasst.

12. Verfahren nach Anspruch 10 oder 11, wobei das Kultivieren in iii. bei einem pH-Wert in dem Bereich von pH 4,8 bis 6,7, im Speziellen bei einer Temperatur in dem Bereich von 15 °C bis 35 °C, vorzugsweise in dem Bereich von 25 °C bis 28 °C, im Speziellen unter aeroben Bedingungen durchgeführt wird, wobei im Speziellen das Kultivieren in iii. zumindest so lange durchgeführt wird, bis die Entwicklung einer roten/purpurfarbenen Farbe in dem Kulturmedium und/oder in der Biomasse festgestellt wird.

13. Verfahren nach einem der Ansprüche 10 bis 12, wobei das Kulturmedium ein flüssiges oder ein festes Medium ist.

14. Verfahren nach einem der Ansprüche 10 bis 13, ferner umfassend die Zugabe von FeCl₃ zur Herstellung eines dunklen Pigments, wobei das Pigment im Speziellen eine Helligkeit (L) in dem Bereich von 0 bis 20 % aufweist.

15. Verfahren zur Extraktion eines Farbstoffs auf Pilzbasis, wobei das Verfahren die aufeinanderfolgenden Schritte umfasst:
i. Trocknen von geernteter Biomasse und/oder geerntetem Kulturmedium, das den Pilz nach Anspruch 1 umfasst;
ii. Suspendieren des getrockneten Materials aus i. in einem Lösungsmittel, vorzugsweise in einem Alkohol, noch bevorzugter in Ethanol;
iii. Verdampfen des Lösungsmittels aus ii.;
iv. Resuspendieren des Rückstands aus iii. in einem Lösungsmittel, das sich von dem Lösungsmittel aus ii. unterscheidet, vorzugsweise in einem Ester, noch bevorzugter in Ethylacetat;
v. Verdampfen des Lösungsmittels aus iv.; und
vi. optional Resuspendieren des Rückstands aus v.

## Revendications

1. Champignon isolé appartenant à l'espèce *Fusarium solani* déposé sous le numéro DSM 34187.

2. Utilisation du champignon isolé de la revendication 1 pour la teinture d'un substrat.

3. Utilisation de la revendication 2, dans laquelle le substrat est un matériau textile choisi dans le groupe constitué par un matériau textile naturel, un matériau textile synthétique et les combinaisons de matériaux textiles naturel et synthétique, en particulier dans laquelle le matériau textile naturel est choisi dans le groupe constitué par le coton, la soie, la laine, l'abaca, le coir, le lin, le chanvre, le bois, le cachemire et le mohair, et dans laquelle le matériau textile synthétique est choisi dans le groupe constitué par le polyester, la rayonne, l'acrylique, le polycarbonate, le polyéthylène, le spandex, l'acétate, le lyocell et le modal.

4. Procédé de changement de la couleur d'un substrat comprenant les étapes séquentielles de :
i. production d'un inoculum du champignon isolé de la revendication 1 ;
ii. inoculation d'un milieu de culture avec l'inoculum ;
iii. préculture éventuelle du milieu de culture inoculé et inactivation éventuelle du champignon après la préculture ;
iv. mise en contact du substrat avec le milieu de culture de ii. ou de iii. jusqu'à ce que la couleur souhaitée soit obtenue, et
v. chauffage du substrat, en particulier dans lequel le chauffage du substrat est effectué à une température comprise dans la plage de 60°C à 121°C, en particulier effectué pendant au moins 20 minutes.

5. Procédé de la revendication 4, dans lequel la préculture en iii. et/ou la mise en contact en iv. est effectuée à un pH compris dans la plage de pH 4,8 à 6,7, en particulier à une température comprise dans la plage de 15°C à 35°C, en particulier dans des conditions aérobies.

6. Procédé de la revendication 4 ou 5, dans lequel le substrat est un matériau textile choisi dans le groupe constitué par un matériau textile naturel, un matériau textile synthétique et les combinaisons de matériaux textiles naturel et synthétique, en particulier dans lequel le matériau textile naturel est choisi dans le groupe constitué par le coton, la soie, la laine, l'abaca, le coir, le lin, le chanvre, le bois, le cachemire et le mohair, et dans lequel le matériau textile synthétique est choisi dans le groupe constitué par le polyester, la rayonne, l'acrylique, le polycarbonate, le polyéthylène, le spandex, l'acétate, le lyocell et le modal.

7. Procédé de l'une quelconque des revendications 4 à 6, dans lequel le milieu de culture comprend une source de glucides, en particulier du glucose, de préférence dans la plage de 1 à 4 % (m/v).

8. Procédé de l'une quelconque des revendications 4 à 7, dans lequel le milieu de culture est un liquide ou un milieu solide.

9. Procédé de l'une quelconque des revendications 4 à 8, comprenant en outre la mise en contact du substrat avec du FeCl₃, dans lequel ladite mise en contact conduit en particulier à une diminution de la luminosité (L) conformément au modèle de couleurs HSL.

10. Procédé de production d'un colorant fongique comprenant les étapes séquentielles de :
i. production d'un inoculum du champignon isolé de la revendication 1 ;
ii. inoculation d'un milieu de culture avec l'inoculum ;
iii. culture du milieu de culture inoculé ;
iv. récolte de la biomasse et/ou du milieu de culture ; et
v. extraction éventuelle du colorant à partir du matériau de iv. récolté.

11. Procédé de la revendication 10, dans lequel le milieu de culture comprend une source de glucides, en particulier du glucose, de préférence dans la plage de 1 à 4 % (m/v).

12. Procédé de la revendication 10 ou 11, dans lequel la culture en iii. est effectuée à un pH compris dans la plage de pH 4,8 à 6,7, en particulier à une température comprise dans la plage de 15°C à 35°C, de préférence dans la plage de 25°C à 28°C, en particulier dans des conditions aérobies, en particulier la culture en iii. est effectuée au moins jusqu'à ce que le développement d'une couleur rouge/violette soit détecté dans le milieu de culture et/ou dans la biomasse.

13. Procédé de l'une quelconque des revendications 10 à 12, dans lequel le milieu de culture est un liquide ou un milieu solide.

14. Procédé de l'une quelconque des revendications 10 à 13, comprenant en outre l'ajout de FeCl₃ pour produire un pigment foncé, en particulier ledit pigment présente une luminosité (L) comprise dans la plage de 0 à 20 %.

15. Procédé d'extraction d'un colorant fongique, ledit procédé comprenant les étapes séquentielles de :
i. séchage de la biomasse récoltée et/ou du milieu de culture récolté comprenant le champignon de la revendication 1 ;
ii. mise en suspension du matériau séché provenant de i. dans un solvant, de préférence dans un alcool, plus préférablement dans l'éthanol.
iii. évaporation du solvant de ii. ;
iv. remise en suspension du résidu de iii. dans un solvant différent du solvant de ii., de préférence dans un ester, plus préférablement dans l'acétate d'éthyle ;
v. évaporation du solvant de iv. ; et
vi. remise en suspension éventuelle du résidu de v..
